# EUROPÄISCHE PATENTSCHRIFT

(11) **EP 4 178 644 B1**
(45) Veröffentlichungstag und Bekanntmachung des Hinweises auf die Patenterteilung: **24.04.2024**
(21) Anmeldenummer: 22741722.7
(22) Anmeldetag: 29.06.2022
(51) Int. Cl.: A61M 5/145

(54) **FÖRDERUNGSABSICHERUNG BEI EINER SPRITZPUMPE**
DELIVERY SAFEGUARD FOR A SYRINGE PUMP
SECURITE D'ADMINISTRAION POUR UN POMPE A SERINGUE

(30) Priorität: 01.07.2021 DE 102021116994
(43) Veröffentlichungstag der Anmeldung: 17.05.2023
(73) Patentinhaber: B. Braun Melsungen AG, 34212 Melsungen (DE)
(72) Erfinder: SCHWARZ, Jan, 34212 Melsungen (DE); FALK, Christian, 36251 Bad Hersfeld (DE); ERLEN, Christoph, 34132 Kassel (DE); MAURO, Christian, 34212 Melsungen (DE); MÖLLER, Sebastian, 37235 Hessisch Lichtenau (DE); SCHÜTZ, Joachim, 36041 Fulda (DE); WOLF, Daniel, 89346 Bibertal (DE)
(74) Vertreter: Winter, Brandl - Partnerschaft mbB
(86) Internationale Anmeldenummer: PCT/EP2022/067886
(87) Internationale Veröffentlichungsnummer: WO 2023/275139

(56) Entgegenhaltungen:
- WO-A1-2016/183342
- US-A- 4 529 401
- US-A1- 2014 188 076

## Beschreibung

### Technisches Gebiet

Die vorliegende Offenbarung betrifft eine redundante Förderungsüberwachung einer medizinischen Fluidpumpe.

### Hintergrund der Erfindung

In der Medizin werden verbreitet Fluidpumpen, insbesondere Spritzenpumpen zur Versorgung eines Patienten mit einer definierten Dosis an Medikamenten eingesetzt. Hierbei ist eine mit einem Medikament aufgezogene Spritze in die einen Grundkörper der Spritzenpumpe eingespannt und ein Spritzenkolben wird mittels eines Antriebskopfes mit einer vorbestimmten Geschwindigkeit in einen Spritzenzylinder eingeschoben. Hierfür wird der Antriebskopf mittels eines Haltearms mit der vorbestimmten Geschwindigkeit in Richtung des Grundkörpers bewegt. Über die vorbestimmte Geschwindigkeit ist eine Medikamentenversorgungsrate einstellbar. Diese vorbestimmte Geschwindigkeit ist durch einen Bediener der Fluidpumpe voreingestellt. Der Antrieb des Antriebskopfes mittels des Haltearms erfolgt über einen Motor, der eine Antriebskraft auf eine Vortriebsspindel überträgt. Die Vortriebsspindel ist mittels eines Vortriebsschlittens mit dem Haltearm verbunden und sorgt dafür, dass sich der Haltearm mit dem Antriebskopf relativ zu dem Grundkörper bewegt. Spritzenpumpen sind beispielsweise aus der EP 0 566 825 A1 bekannt.

Da die Geschwindigkeit, mit welcher der Spritzenkolben in den Spritzenzylinder eingeschoben wird, sehr gering sein kann und eine Medikamentenapplikation mittels der Spritzenpumpe über einen längeren Zeitraum erfolgt, ist es notwendig, sicherzustellen, dass die Medikamentenapplikation störungsfrei abläuft.

US 2014 / 188 076 A1 offenbart eine Spritzenpumpe und einem Motor, welche den Antriebskopf über eine Spindel antreibt und eine Bewegungsdetektionseinheit in Form eines magnetischen Drehencoders an dem Motor und einem linearen Positionssensor.

WO 2016 / 183 342 A1 offenbart eine Spritzenpumpe mit redundant ausgebildeten Linearpotentiometern zur Überwachung einer Verfahrbewegung des Antriebskopfes.

### Kurzbeschreibung der Erfindung

Die Aufgabe der vorliegenden Erfindung ist es also, ein System bereitzustellen, welches gewährleistet, dass eine Störung des Verfahrens des Antriebskopfes und damit eine Störung der Medikamentenapplikation zuverlässig detektiert wird.

Diese Aufgabe wird durch eine erfindungsgemäße medizinische Fluidpumpe gemäß Anspruch 1, insbesondere eine Spritzenpumpe mit redundanter Förderungsüberwachung gelöst.

Die medizinische Fluidpumpe, insbesondere Spritzenpumpe, beinhaltet ein Gehäuse und einen linear mittels eines Haltearms (Schubstange) zu dem Gehäuse bewegbaren/verfahrbaren Antriebskopf, welcher von einem Motor angetrieben wird, wobei die Spritzenpumpe mit mindestens einer ersten Bewegungsdetektionseinheit zur Erfassung einer ersten Bewegungsart (Linearbewegung) und mindestens einer zweiten Bewegungsdetektionseinheit zur Erfassung einer zweiten Bewegungsart (Rotationsbewegung) unterschiedlich zur ersten Bewegungsart und insgesamt, d.h. im Zusammenspiel zur sicheren Detektion einer Bewegung des Antriebskopfes versehen ist. Die medizinische Fluidpumpe gibt einen Alarm aus, wenn die erste Bewegungsdetektionseinheit und/oder die zweite Bewegungsdetektionseinheit eine Unterbrechung des Verfahrens des Antriebskopfes detektiert. Die erste Bewegungsdetektionseinheit ist als ein optischer Drehcoder mit einer Schlitzscheibe und einer Lichtschranke ausgebildet.

In anderen Worten weist die Fluidpumpe einen Grundkörper in Form des Gehäuses auf, der vorgesehen und ausgebildet ist, eine Spritze, insbesondere einen Spritzenzylinder aufzunehmen. Weiterhin weist die Fluidpumpe den Antriebskopf auf, der an einem Endabschnitt des Haltearms ausgebildet oder montiert ist. Der Haltearm ist linear zu dem Gehäuse der Fluidpumpe mittels des Motors beweglich, sodass der Antriebskopf in Relation zu dem Gehäuse verfahrbar ist. Eine Kolbenplatte des Spritzenkolbens ist in dem Antriebskopf fixiert/gehalten. Durch ein Verfahren des Antriebskopfes und damit des mit dem Antriebskopf gekoppelten/koppelbaren Spritzenkolbens relativ zu dem Gehäuse und damit relativ zu dem Spritzenzylinder mit einer vorbestimmten Geschwindigkeit wird ein Medikament mit einer vorbestimmten Medikamentenversorgungsrate/Medikamentenapplikationsrate (Medikamentenvolumen pro Zeiteinheit) an den Patienten appliziert. Die mindestens eine erste Bewegungsdetektionseinheit detektiert (direkt) das Verfahren des Antriebskopfes relativ zu dem Gehäuse. Ebenso detektiert die mindestens eine zweite Bewegungsdetektionseinheit (indirekt) das Verfahren des Antriebskopfes relativ zu dem Gehäuse.

Durch die mindestens eine erste Bewegungsdetektionseinheit und die mindestens eine zweite Bewegungsdetektionseinheit ist sichergestellt, dass selbst bei einem Ausfall der ersten Bewegungsdetektionseinheit oder der zweiten Bewegungsdetektionseinheit eine Unterbrechung des Verfahrens des Antriebskopfes relativ zu dem Gehäuse detektiert werden kann und ein Alarm ausgegeben werden kann. Somit ist sichergestellt, dass auch im Falle eines Ausfalls einer der Bewegungsdetektionseinheiten eine Unterbrechung in der Medikamentenapplikation zuverlässig durch einen Alarm an einen Bediener ausgegeben wird. Außerdem ist sichergestellt, dass eine Unterbrechung des Kraftflusses zwischen Motor und Haltearm erfasst werden kann, beispielsweise indem eine Detektionseinheit zwar eine Bewegung des Motors erkennt, die andere Detektionseinheit ein Ruhen des Haltearms erfasst.

In einem ersten bevorzugten Aspekt sind die erste Bewegungsdetektionseinheit und die zweite Bewegungsdetektionseinheit als divers redundante Bewegungsdetektionseinheiten vorgesehen und ausgebildet.

In anderen Worten liegen die Bewegungsdetektionseinheiten in diversitärer Redundanz vor. In nochmals anderen Worten basieren die erste Bewegungsdetektionseinheit und die zweite Bewegungsdetektionseinheit auf unterschiedlichen Wirkprinzipien und/oder sind an unterschiedlichen Baugruppen und/oder in unterschiedlicher Ausrichtung in der Fluidpumpe ausgebildet und/oder sind von unterschiedlichen Herstellern und/oder sind unterschiedlichen Typen.

Das Vorsehen der ersten Bewegungsdetektionseinheit und der zweiten Bewegungsdetektionseinheit in diversitärer Redundanz reduziert das Risiko, dass beide Bewegungsdetektionseinheiten aufgrund eines einzelnen Ereignisses ausfallen und erhöht damit die Detektionssicherheit des Ausfalls der ersten Bewegungsdetektionseinheit oder der zweiten Bewegungsdetektionseinheit.

In einem weiteren bevorzugten Aspekt ist die erste Bewegungsdetektionseinheit an dem Motor vorgesehen und ausgebildet.

In anderen Worten ist die erste Bewegungsdetektionseinheit an einem Rotor des Motors vorgesehen und ausgebildet. Die erste Bewegungsdetektionseinheit detektiert die Rotation des Rotors in einem Stator des Motors. Durch die Detektion der Bewegung direkt an dem Motor kann sichergestellt werden, dass durch den Motor eine Antriebskraft zur Verfügung gestellt wird.

In einem weiteren bevorzugten Aspekt ist die erste Bewegungsdetektionseinheit an einer Vortriebsspindel vorgesehen und ausgebildet, die eine Antriebskraft von dem Motor auf den Haltearm überträgt.

In anderen Worten ist die erste Bewegungsdetektionseinheit an der Vortriebsspindel ausgebildet und detektiert die Rotation der Vortriebsspindel. Durch die Detektion der Bewegung an der Vortriebsspindel kann sichergestellt werden, dass die durch den Motor erzeugte Antriebskraft die Vortriebsspindel in Rotation versetzt.

In einem weiteren bevorzugten Aspekt ist die zweite Bewegungsdetektionseinheit an dem Haltearm vorgesehen und ausgebildet.

In andern Worten detektiert die zweite Bewegungsdetektionseinheit direkt die lineare Bewegung des Haltearms und damit des an dem Haltearm ausgebildeten Antriebskopf zu dem Gehäuse der Fluidpumpe. Die direkte Detektion der Relativbewegung zwischen Antriebskopf und Gehäuse gewährleistet, dass die für die Medikamentenapplikation notwendige Bewegung tatsächlich erfolgt.

In einem weiteren bevorzugten Aspekt ist die erste Bewegungsdetektionseinheit als Drehcoder an dem Motor oder der Vortriebsspindel vorgesehen und ausgebildet.

In anderen Worten ist in einer Ausführungsform, in der die erste Bewegungsdetektionseinheit an dem Motor vorgesehen und ausgebildet ist, der Drehcoder verdrehsicher an dem Rotor des Motors ausgebildet. In einer weiteren Ausführungsform, in der die erste Bewegungsdetektionseinheit an der Vortriebsspindel vorgesehen und ausgebildet ist, ist der Drehcoder verdrehsicher an der Vortriebsspindel ausgebildet. Bei dem Drehcoder handelt es sich um einen optischen Drehcoder mit einer Schlitzscheibe und einer Lichtschranke. Die Lichtschranke beinhaltet mindestens eine Lichtquelle, beispielsweise in Form einer LED, und mindestens einen Lichtdetektor, beispielsweise in Form eines Fototransistors. Die Schlitzscheibe ist verdrehsicher an einem Bauteil befestigt, dessen Bewegung detektiert wird. Die Lichtquelle emittiert ein Licht in Richtung des Lichtdetektors. Die Lichtschranke ist dabei so angeordnet, dass das Licht durch Schlitze der Schlitzscheibe emittiert wird. Bei einer Rotation der Schlitzscheibe unterbrechen ungeschlitzte Abschnitte der Schlitzscheibe einen Lichtweg zwischen Lichtquelle und Lichtdetektor. Diese Unterbrechungen werden von dem Lichtdetektor detektiert und als Bewegung des Bauteils ausgewertet.

Weitere alternativ vorstellbare Ausprägungsformen des Drehcoders beinhalten einen magnetischen Encoder, einen kapazitiven Encoder oder einen mechanischen Encoder.

Der Drehcoder ermöglicht auf einfache Art, eine Rotationsbewegung des Motors bzw. des Rotors des Motors oder der Vortriebsspindel zu detektieren. Der Drehcoder benötigt einen geringen Bauraum und ist insbesondere in einem geschlossenen Gehäuse störunanfällig.

In einem weiteren bevorzugten Aspekt ist die zweite Bewegungsdetektionseinheit als Längenpotenziometer an dem Haltearm vorgesehen und ausgebildet.

In anderen Worten ist das Längenpotenziometer/Linearpotenziometer so zwischen dem Haltearm und dem Gehäuse ausgebildet, dass es eine Relativbewegung zwischen dem Haltearm und dem Gehäuse detektiert. Vorzugsweise ist ein Lesekopf/Mitnehmerschlitten des Längenpotenziometers an dem Haltearm vorgesehen und ausgebildet. An dem Gehäuse ist ein Weggeber vorgesehen und ausgebildet. Wenn der Haltearm relativ zu dem Gehäuse verfährt, verfährt der fest mit dem Haltearm verbundene Lesekopf relativ zu dem mit dem Gehäuse verbundenen Weggeber und kann so eine Relativbewegung zwischen Haltearm und Gehäuse detektieren. Der Weggeber kann auch an einem fest mit dem Gehäuse verbundenen Bauteil ausgebildet sein. Alternativ ist der Weggeber an dem Haltearm ausgebildet und der Lesekopf ist an dem Gehäuse ausgebildet.

Vorzugsweise handelt es sich bei dem Längenpotenziometer um ein Längenpotenziometer auf Basis der Leitplastiktechnologie. Alternativ kann es sich bei dem Längenpotenziometer um ein Folienpotenziometer oder jede andere Art von Längenpotenziometer handeln, das geeignet ist, eine lineare Bewegung zu detektieren.

Vorzugsweise kann das Längenpotenziometer eine Position des Haltearms zu dem Gehäuse bestimmen. In anderen Worten ist das Längenpotenziometer vorgesehen und ausgebildet, neben einer Relativbewegung zwischen Gehäuse und Haltearm einen Positionswert zwischen Haltearm und Gehäuse zu bestimmen. So kann festgestellt werden, in welcher Position sich der Haltearm relativ zu dem Gehäuse befindet. Zusätzlich ist so eine Detektion des Verfahrweges möglich, was es der medizinischen Fluidpumpe unter Verwendung weiterer Daten wie einem Spritzenquerschnitt ermöglicht, eine applizierte Medikamentenmenge anhand des Verfahrwegs des Haltearms relativ zu dem Gehäuse zu berechnen.

In einem weiteren bevorzugten Aspekt ist die zweite Bewegungsdetektionseinheit als Längenpotenziometer an einem Vortriebsschlitten vorgesehen und ausgebildet, wobei es sich bei dem Vortriebsschlitten um ein Verbindungsglied zwischen Vortriebsspindel und Haltearm handelt.

In anderen Worten ist der Lesekopf/Mitnehmerschlitten des Längenpotenziometers an dem Vortriebsschlitten vorgesehen und ausgebildet, der eine Antriebskraft/ein Antriebsmoment des Motors in eine lineare Bewegung des Haltearms umsetzt. Der Vortriebsschlitten ist fest mit dem Haltearm verbunden. An dem Gehäuse ist der Weggeber vorgesehen und ausgebildet. Wenn der Haltearm mit dem fest mit dem Haltearm verbundenen Vortriebsschlitten relativ zu dem Gehäuse verfährt, verfährt der fest mit dem Vortriebsschlitten verbundene Lesekopf relativ zu dem mit dem Gehäuse verbundenen Weggeber und kann so eine Relativbewegung zwischen Haltearm und Gehäuse detektieren. Der Weggeber kann auch an einem fest mit dem Gehäuse verbundenen Bauteil ausgebildet sein.

In einem weiteren bevorzugten Aspekt ist die erste Bewegungsdetektionseinheit an einem Getriebeelement vorgesehen und ausgebildet, dass die Antriebskraft von dem Motor auf die Vortriebsspindel überträgt.

In anderen Worten ist zwischen dem Motor und der Vortriebsspindel ein Getriebe/eine Übersetzung vorgesehen und ausgebildet, das/die ein Drehzahl/Drehmomentverhältnis zwischen Motor und Vortriebsspindel umsetzt. Vorzugsweise beinhaltet das Getriebe zumindest zwei Zahnräder. An einem der Zahnräder ist vorzugsweise die zweite Bewegungsdetektionseinheit, insbesondere als Drehencoder vorgesehen und ausgebildet.

In einem weiteren bevorzugten Aspekt ist die medizinische Fluidpumpe mit einer Steuerungsvorrichtung ausgebildet, die ein von der ersten Bewegungsdetektionseinheit erfasstes ersten Bewegungssignal mit einem von der zweiten Bewegungsdetektionseinheit detektierten zweiten Bewegungssignal vergleicht und bei einer Abweichung von einem in der Steuerungsvorrichtung hinterlegten Sollverhältnis zwischen dem ersten Bewegungssignal und dem zweiten Bewegungssignal den Alarm ausgibt.

In einem weiteren bevorzugten Aspekt ist das Sollverhältnis ein Drehzahl/Weg-Verhältnis.

Anders ausgedrückt ist die Steuervorrichtung in dem bevorzugten Aspekt ausgebildet, die von der ersten Bewegungsdetektionseinheit erfasste Drehzahl mit der von der zweiten Bewegungsdetektionseinheit erfassten Längenänderung in ein Verhältnis zu setzen. Bei dem Verhältnis handelt es sich um ein konstantes Verhältnis. Die Steuervorrichtung vergleicht das Verhältnis mit dem hinterlegten Sollverhältnis und gibt bei einer Abweichung bzw. wenn ein Detla zwischen Verhältnis und Sollverhältnis einen Grenzwert überschreitet, einen Alarm aus.

Bei dem Alarm kann es sich um einen optischen, akustischen und/oder haptischen Alarm handeln. Der Alarm kann von der medizinischen Fluidpumpe beispielsweise über einen Lautsprecher und/oder eine Anzeigevorrichtung ausgegeben werden. Bei der Anzeigevorrichtung kann es sich um einen Display, eine oder mehrere LEDs oder dergleichen handeln.

Alternativ oder zusätzlich kann die medizinische Fluidpumpe den Alarm als ein Alarmsignal in Form eines Funksignals an eine zentrale Steuereinrichtung und/oder ein mobiles Handgerät oder dergleichen übermitteln.

Der Alarm kann verschiedene Ausprägungen aufweisen. Beispielsweise kann der Alarm bei einer geringfügigen Abweichung des Verhältnisses von dem Sollverhältnis eine geringere Ausprägung aufweisen als bei einer hohen Abweichung des Verhältnisses von dem Sollverhältnis.

Die Steuervorrichtung kann optional bei Auslösen des Alarms den Motor und damit eine Zuführung eines Medikaments an einen Pateinten unterbrechen.

### Kurzbeschreibung der Figuren

Fig. 1 ist eine Darstellung einer erfindungsgemäßen medizinischen Fluidpumpe in Form einer Spritzenpumpe.
Fig. 2 ist eine Darstellung einer Innenseite einer Oberschale eines Gehäuses der medizinischen Fluidpumpe.
Fig. 3 ist eine Darstellung eines Längenpotenziometers an einem Antriebsstrang.

### Detaillierte Figurenbeschreibung

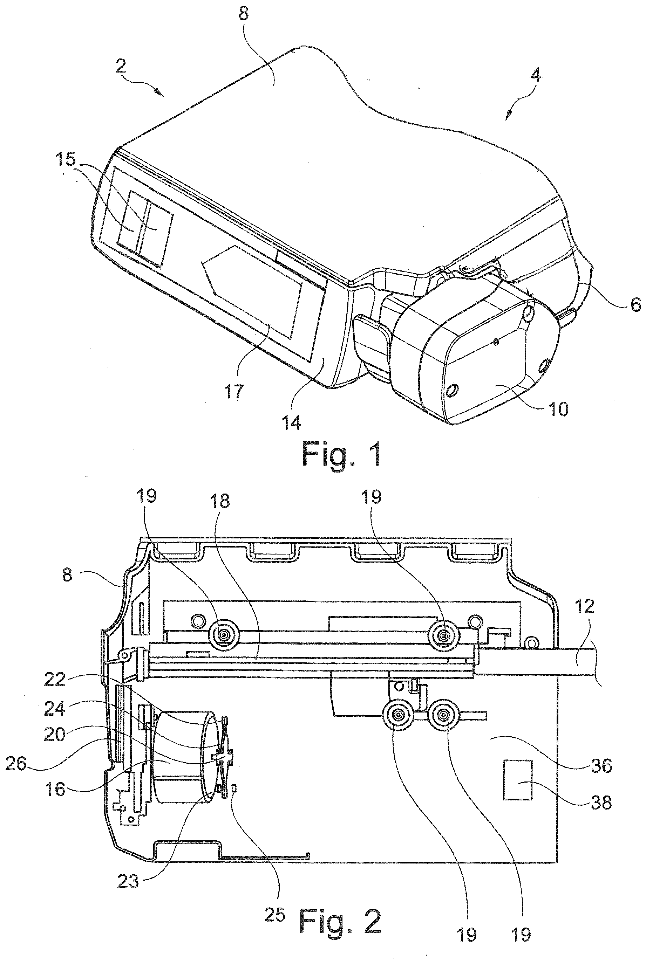

Fig. 1 zeigt eine erfindungsgemäße medizinische Fluidpumpe, insbesondere eine Spritzenpumpe 2. Die grundlegende Funktionsweise der Spritzenpumpe 2 ist in der EP 0 566 825 A1 ausreichend beschrieben, sodass hier auf eine detaillierte Beschreibung der Spritzenpumpe 2 und ihrer grundlegenden Funktionen verzichtet wird.

Die in Fig. 1 dargestellte Spritzenpumpe 2 beinhaltet ein Gehäuse 4 mit einer Gehäuseunterschale 6 und einer Gehäuseoberschale 8. Ein Antriebskopf 10 ist über einen Haltearm 12 (siehe Fig. 2) relativ zu dem Gehäuse 4 beweglich mit der Spritzenpumpe 2 verbunden. An der Vorderseite der Spritzenpumpe 2 ist eine Frontklappe 14 vorgesehen und ausgebildet. Die Frontklappe 14 ist klappbar über Scharniere an der Gehäuseunterschale 6 angelenkt und beinhaltet Bedienelemente 15 und ein Display 17.

Fig. 2 zeigt eine Innenseite der Gehäuseoberschale 8 der erfindungsgemäßen Spritzenpumpe 2. Der Haltearm 12 wird von dem Motor 16 über einen Antriebsstrang 18 relativ zu dem Gehäuse 4 bewegt. Der Antriebsstrang ist mittels Schrauben 19 befestigt. Der Motor 16 weist an einem Rotor verdrehsicher angebracht eine Schlitzscheibe 20 auf. Die Schlitzscheibe 20 beinhaltet Flügel 22 und Schlitze 24. Ein von einer Lichtquelle 23 der Lichtschranke emittierter Lichtstrahl wird bei einer Rotation des Rotors durch die Flügel 22 der Schlitzscheibe 20 abwechselnd unterbrochen und nicht unterbrochen. Somit kann über ein von einem Lichtdetektor 25 der Lichtschranke detektiertes Signal eine Rotation der Schlitzscheibe 20 und damit die Rotation des Rotors des Motors 16 detektiert werden. Die Schlitzscheibe 20 und die Lichtschranke sind Bestandteil einer ersten Bewegungsdetektionseinheit der Spritzenpupe 2.

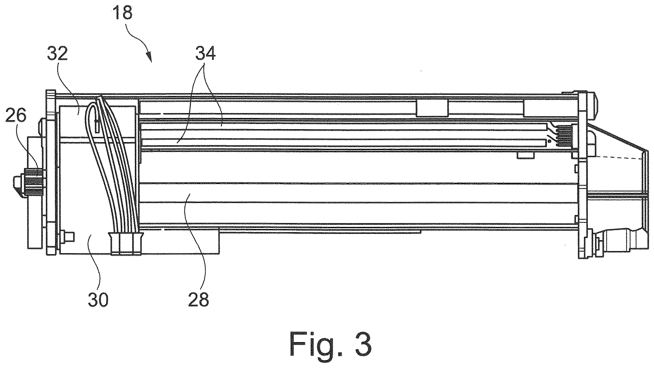

Fig.3 zeigt eine Innenansicht des Antriebsstrangs 18. Über ein Getriebe 26 wird eine von dem Motor 16 erzeugte Antriebskraft/ein von dem Motor 16 erzeugtes Antriebsmoment auf eine Vortriebsspindel 28 übertragen. Ein fest mit dem Haltearm 12 verbundener Vortriebsschlitten 30 kämmt mit der Vortriebsspindel 28. Die von dem Motor 16 erzeugte Antriebskraft ist so in der Lage, den Haltearm relativ zu dem Gehäuse zu bewegen. An dem Vortriebsschlitten 30 ist der Lesekopf 32 vorgesehen und ausgebildet. Der Lesekopf ist dabei fest mit dem Vortriebsschlitten 30 verbunden. An der Gehäuseoberschale 8 ist ein Weggeber 34 vorgesehen und ausgebildet. Der Weggeber 34 ist fest mit der Gehäuseoberschale 8 verbunden. Alternativ kann der Weggeber 34 an einem beliebigen Bauteil der Spritzenpumpe 2 befestigt sein, das fest mit dem Gehäuse 4 der Spritzenpumpe 2 verbunden ist.

Wenn von dem Motor 16 eine Antriebskraft auf das Getriebe 26 ausgeübt wird, wird die Antriebsspindel 28 in Rotation versetzt. Die Antriebskraft wird über den Vortriebsschlitten 30 auf den Haltearm 12 übertragen und der Haltearm 12 bewegt sich relativ zu dem Gehäuse 4 der Spritzenpumpe 2. Der fest mit dem Vortriebsschlitten verbundene Lesekopf 32 verfährt/bewegt sich dabei relativ zu dem fest an dem Gehäuse ausgebildeten Weggeber 34. Der Lesekopf 32 detektiert die Bewegung relativ zu dem Weggeber 34.

Vorzugsweise sind auf dem Weggeber 34 Positionen kodiert, so dass der Lesekopf 32 neben der Bewegung auch die Position des Lesekopfes 32 relativ zu dem Weggeber 34 detektiert. Bei dem Lesekopf 32 und dem Weggeber 34 sind Bestandteil der zweiten Bewegungsdetektionseinheit der Spritzenpumpe 2.

Auf einer Hauptplatine 36 (siehe Fig. 2) ist eine Berechnungseinheit 38 vorgesehen und ausgebildet. Die Berechnungseinheit 38 erhält von der ersten Bewegungsdetektionseinheit in Form von der Schlitzscheibe 20 und der Lichtschranke, bestehend aus der Lichtquelle 23 und dem Lichtdetektor 25, ein erstes Bewegungssignal und von der zweiten Bewegungsdetektionseinheit in Form von dem Lesekopf 32 und dem Weggeber 34 ein zweites Bewegungssignal. Die Berechnungseinheit 38 gleicht das erste Bewegungssignal mit dem zweiten Bewegungssignal anhand eines in der Berechnungseinheit 38 hinterlegten Kennfelds ab. Wenn von der Berechnungseinheit 38 festgestellt wird, dass das von der ersten Bewegungsdetektionseinheit erhaltene erste Bewegungssignal nicht zu dem von der zweiten Bewegungsdetektionseinheit erhaltenen zweiten Bewegungssignal passt, veranlasst die Berechnungseinheit 38 ein optische und/oder akustische Alarmausgabe.

### Bezugszeichenliste

- 2: Spritzenpumpe
- 4: Gehäuse
- 6: Gehäuseunterschale
- 8: Gehäuseoberschale
- 10: Antriebskopf
- 12: Haltearm
- 14: Frontklappe
- 15: Bedienelemente
- 16: Motor
- 17: Display
- 18: Antriebsstrang
- 19: Schraube
- 20: Schlitzscheibe
- 22: Flügel
- 23: Lichtquelle
- 24: Schlitze
- 25: Lichtdetektor
- 26: Getriebe
- 28: Vortriebsspindel
- 30: Vortriebsschlitten
- 32: Lesekopf
- 34: Weggeber
- 36: Platine
- 38: Berechnungseinheit

## Patentansprüche

1. Medizinische Fluidpumpe, insbesondere Spritzenpumpe (2), mit einem Gehäuse (4) und einem linear mittels einem Haltearm (12) zu dem Gehäuse (4) bewegbaren Antriebskopf (10), welcher von einem Motor (16) angetrieben wird, wobei die Fluidpumpe mit mindestens einer ersten Bewegungsdetektionseinheit (25) und mindestens einer zweiten Bewegungsdetektionseinheit zur Detektion einer Bewegung des Antriebskopfes (10) versehen ist und die medizinische Fluidpumpe einen Alarm ausgibt, wenn die erste Bewegungsdetektionseinheit (25) und/oder die zweite Bewegungsdetektionseinheit eine Unterbrechung des Verfahrens des Antriebskopfes detektiert **dadurch gekennzeichnet, dass** die erste Bewegungsdetektionseinheit (25) als ein optischer Drehcoder mit einer Schlitzscheibe (20) und einer Lichtschranke (23; 25) ausgebildet ist.

2. Medizinische Fluidpumpe nach Anspruch 1, **dadurch gekennzeichnet, dass** die erste Bewegungsdetektionseinheit (25) und die zweite Bewegungsdetektionseinheit als divers redundante Bewegungsdetektionseinheiten vorgesehen und ausgebildet sind.

3. Medizinische Fluidpumpe nach Anspruch 1 oder 2, **dadurch gekennzeichnet, dass** die erste Bewegungsdetektionseinheit (25) an dem Motor (16) vorgesehen und ausgebildet ist.

4. Medizinische Fluidpumpe nach Anspruch 1 oder 2, **dadurch gekennzeichnet, dass** die erste Bewegungsdetektionseinheit (25) an einer Vortriebsspindel (28) vorgesehen und ausgebildet ist, die eine Antriebskraft von dem Motor (16) auf den Haltearm (12) überträgt.

5. Medizinische Fluidpumpe nach einem der Ansprüche 1 bis 4, **dadurch gekennzeichnet, dass** die zweite Bewegungsdetektionseinheit an dem Haltearm (12) vorgesehen und ausgebildet ist.

6. Medizinische Fluidpumpe nach Anspruch 5, **dadurch gekennzeichnet, dass** die zweite Bewegungsdetektionseinheit als Längenpotenziometer an dem Haltearm (12) vorgesehen und ausgebildet ist.

7. Medizinische Fluidpumpe nach einem der Ansprüche 1 bis 4, **dadurch gekennzeichnet, dass** die zweite Bewegungsdetektionseinheit als Längenpotenziometer an einem Vortriebsschlitten (30) vorgesehen und ausgebildet ist, wobei es sich bei dem Vortriebsschlitten (30) um ein Verbindungsglied zwischen Vortriebsspindel (28) und Haltearm (12) handelt.

8. Medizinische Fluidpumpe nach einem der Ansprüche 1 oder 2, **dadurch gekennzeichnet, dass** die erste Bewegungsdetektionseinheit (25) an einem Getriebeelement vorgesehen und ausgebildet ist, dass die Antriebskraft von dem Motor (16) auf die Vortriebsspindel (28) überträgt.

9. Medizinische Fluidpumpe nach einem der Ansprüche 1 bis 8, **gekennzeichnet durch** eine Steuerungsvorrichtung, die ein von der ersten Bewegungsdetektionseinheit (25) erfasstes ersten Bewegungssignal mit einem von der zweiten Bewegungsdetektionseinheit detektierten zweiten Bewegungssignal vergleicht und bei einer Abweichung von einem in der Steuerungsvorrichtung hinterlegten Sollverhältnis zwischen dem ersten Bewegungssignal und dem zweiten Bewegungssignal den Alarm ausgibt.

10. Medizinische Fluidpumpe nach Anspruch 9, wobei das Sollverhältnis ein Drehzahl/Weg-Verhältnis ist.

11. Medizinische Fluidpumpe nach Anspruch 6, **dadurch gekennzeichnet, dass** die zweite Bewegungsdetektionseinheit ein Längenpotenziometer auf Basis der Leitplastiktechnologie ist.

## Claims

1. A medical fluid pump, in particular syringe pump (2), having a housing (4) and a drive head (10) which can be moved linearly via a retaining arm (12) to the housing (4) and is driven by a motor (16), wherein the fluid pump is provided with at least one first movement detection unit (25) and at least one second movement detection unit for detecting a movement of the drive head (10), and the medical fluid pump emitting an alarm, when the first movement detection unit (25) and/or the second movement detection unit detects an interruption in the movement of the drive head, **characterized in that** the first movement detection unit (25) is configured as an optical rotary encoder with a slotted disk (20) and a light barrier (23; 25).

2. The medical fluid pump according to claim 1, **characterized in that** the first movement detection unit (25) and the second movement detection unit are provided and configured as diverse redundant movement detection units.

3. The medical fluid pump according to claim 1 or 2, **characterized in that** the first movement detection unit (25) is provided and configured on the motor (16).

4. The medical fluid pump according to claim 1 or 2, **characterized in that** the first movement detection unit (25) is provided and configured on an advancing spindle (28) which transmits a driving force from the motor (16) to the retaining arm (12).

5. The medical fluid pump according to one of claims 1 to 4, **characterized in that** the second movement detection unit is provided and configured on the retaining arm (12).

6. The medical fluid pump according to claim 5, **characterized in that** the second movement detection unit is provided and configured as a length potentiometer on the retaining arm (12).

7. The medical fluid pump according to one of claims 1 to 4, **characterized in that** the second movement detection unit is provided and configured as a length potentiometer on an advancing carriage (30), wherein the advancing carriage (30) is a connecting link between advancing spindle (28) and retaining arm (12).

8. The medical fluid pump according to one of claims 1 or 2, **characterized in that** the first movement detection unit (25) is provided and configured on a transmission element that transmits the driving force from the motor (16) to the advancing spindle (28).

9. The medical fluid pump according to one of claims 1 to 8, **characterized by** a control device which compares a first movement signal detected by the first movement detection unit (25) with a second movement signal detected by the second movement detection unit and outputs the alarm in the event of a deviation from a target ratio between the first movement signal and the second movement signal stored in the control device.

10. The medical fluid pump according to claim 9, wherein the target ratio is a speed/displacement ratio.

11. The medical fluid pump according to claim 6, **characterized in that** the second movement detection unit is a length potentiometer based on conductive plastic technology.

## Revendications

1. Pompe à fluide médical, en particulier pompe à seringue (2) comprenant un boîtier (4) et une tête d'entraînement (10) pouvant se déplacer linéairement par rapport au boîtier (4) au moyen d'un bras de retenue (12), qui est entraînée par un moteur (16), dans laquelle la pompe à fluide est pourvue d'au moins une première unité de détection de mouvement (25) et d'au moins une seconde unité de détection de mouvement pour détecter un mouvement de la tête d'entraînement (10) et la pompe à fluide médical émet une alarme lorsque la première unité de détection de mouvement (25) et/ou la seconde unité de détection de mouvement détecte(-ent) une interruption du procédé de la tête d'entraînement, **caractérisée en ce que** la première unité de détection de mouvement (25) est conçue comme un codeur rotatif optique avec un disque à fente (20) et une barrière lumineuse (23 ; 25).

2. Pompe à fluide médical selon la revendication 1, **caractérisée en ce que** la première unité de détection de mouvement (25) et la seconde unité de détection de mouvement sont prévues et conçues comme unités de détection de mouvement diversement redondantes.

3. Pompe à fluide médical selon la revendication 1 ou 2, **caractérisée en ce que** la première unité de détection de mouvement (25) est prévue et conçue au niveau du moteur (16).

4. Pompe à fluide médical selon la revendication 1 ou 2, **caractérisée en ce que** la première unité de détection de mouvement (25) est prévue et conçue au niveau d'une broche de propulsion (28) qui transmet une force d'entraînement du moteur (16) au bras de retenue (12).

5. Pompe à fluide médical selon l'une quelconque des revendications 1 à 4, **caractérisée en ce que** la seconde unité de détection de mouvement est prévue et conçue au niveau du bras de retenue (12).

6. Pompe à fluide médical selon la revendication 5, **caractérisée en ce que** la seconde unité de détection de mouvement est prévue et conçue comme un potentiomètre de longueur au niveau du bras de retenue (12).

7. Pompe à fluide médical selon l'une quelconque des revendications 1 à 4, **caractérisée en ce que** la seconde unité de détection de mouvement est prévue et conçue comme un potentiomètre de longueur au niveau d'un chariot de propulsion (30), dans laquelle le chariot de propulsion (30) est un élément de connexion entre la broche de propulsion (28) et le bras de retenue (12).

8. Pompe à fluide médical selon l'une quelconque des revendications 1 ou 2, **caractérisée en ce que** la première unité de détection de mouvement (25) est prévue et conçue au niveau d'un élément d'engrenage qui transmet la force d'entraînement du moteur (16) à la broche de propulsion (28).

9. Pompe à fluide médical selon l'une quelconque des revendications 1 à 8, **caractérisée par** un dispositif de commande qui compare un premier signal de mouvement détecté par la première unité de détection de mouvement (25) à un second signal de mouvement détecté par la seconde unité de mouvement et qui déclenche l'alarme en cas d'écart par rapport à un rapport de consigne entre le premier signal de mouvement et le second signal de mouvement enregistré dans le dispositif de commande.

10. Pompe à fluide médical selon la revendication 9, dans laquelle le rapport de consigne est un rapport vitesse de rotation/course.

11. Pompe à fluide médical selon la revendication 6, **caractérisée en ce que** la seconde unité de détection de mouvement est un potentiomètre de longueur sur la base de la technologie des plastiques conducteurs.
